# EUROPEAN PATENT SPECIFICATION

(11) **EP 0 466 790 B1**
(45) Date of publication and mention of the grant of the patent: **15.07.1998**
(21) Application number: 90906273.9
(22) Date of filing: 30.03.1990
(51) Int. Cl.: G01N 25/08

(54) **INDICATING DEVICE**
ANZEIGEVORRICHTUNG
DISPOSITIF D'INDICATION

(30) Priority: 08.04.1989 GB 8907948
(43) Date of publication of application: 22.01.1992
(73) Proprietor: ALBA DIAGNOSTICS LIMITED, Glenrothes Fife KY6 2DA (GB)
(72) Inventor: BUCHANAN, Nigel Liquid Levers (Innovations) Ltd, Fife KY8 5TF (GB)
(74) Representative: Szczuka, Jan Tymoteusz
(86) International application number: GB9000481
(87) International publication number: WO9012311

(56) References cited:
- WO-A-87/04249
- US-A- 4 484 822
- IBM Technical Disclosure Bulletin, Volume 13, No. 8, January 1971, N.G. AAKALU et al.: "Float Type Boiling Point Sensor", page 2402

## Description

The present invention relates to an indicating device for indicating the boiling point of fluids especially hygroscopic fluids such as hydraulic fluids, for example automotive brake and clutch fluid.

Brake fluid as used in the hydraulic braking systems of motor-cars and vans is hygroscopic ie is capable of absorbing moisture, and this can have an adverse effect on braking performance especially in the long term. In particular, the absorption of moisture in the brake fluid causes a lowering of the boiling point of the fluid and very possibly to such a degree that temperature increase in the braking system due for example to heavy braking or some fault condition can result in the brake fluid boiling and as will be appreciated this has a very deleterious effect on braking. Therefore it is beneficial to be aware of a serious lowering of the boiling point of brake fluid so that steps can be taken to change the faulty fluid.

A method and device for determining the boiling point of hygroscopic liquids, such as automotive brake and clutch fluid, is described and claimed in EP-A-0074415 (corresponding to US Patent 4484823). The method of EP-A-0074415 involves the heating of the liquid to be tested using a heater immersed in the liquid until the liquid evaporates and gas bubbles rise along the heater, the temperature of the heater effected by the gas bubbles being measured whence the boiling point of the liquid can be ascertained. A further pressure fluid boiling point testing device is described in GB-A-2139763, the device of GB-A-2139763 including a heater for fluid which also serves as a temperature sensor, the device relying on the formation of gas bubbles on the heater for a boiling point testing operation. These prior art devices have the disadvantage of not being particularly accurate and/or forming noxious vapours due to the evaporation of hydraulic fluid.

It is an object of the present invention to provide an apparatus for indicating the boiling point of brake fluid which avoids or reduces one or more of these disadvantages.

According to the present invention there is provided an apparatus for use in indicating the boiling point of fluid especially a hygroscopic fluid such as hydraulic fluid, and comprising a meter of the portable hand held type formed and arranged for insertion of a probe portion thereof substantially vertically into a body of fluid in a fluid reservoir for fluid testing, heating means being provided in the probe portion for heating fluid in which the probe portion is disposed, said meter including temperature monitoring means for monitoring the temperature rise of fluid heated by said heating means for use in indicating the boiling point temperature of the fluid, said probe portion including a casing defining a chamber located at the lower end of the probe portion so as to be substantially immersed in said body of fluid when said probe portion is inserted thereinto for holding a portion of said body of fluid within the casing said heating means being located in said chamber and said probe portion having upper and lower fluid port means which exit below a fluid fill level in said chamber for permitting flow of fluid to and from said chamber at upper and lower end portions thereof so that a said portion of said body of fluid can be semi-encapsulated in said chamber, and the temperature monitoring means having a sensor disposed in said chamber so as to be substantially immersed in said semi-encapsulated fluid portion for monitoring the change in temperature thereof when heated by said heating means;
characterized in that said probe portion includes a bottom closure wall defining the lower fluid port means so that the chamber is substantially enclosed within the probe portion, and said upper and lower fluid port means are of a substantially restricted form for substantially preventing flow of fluid to and from said chamber during heating of said fluid up to its boiling point.

In a further aspect the present invention provides a method of determining the boiling point of fluid especially a hygroscopic fluid such as hydraulic fluid, which method comprises the steps of: providing an apparatus according to the invention and comprising a portable hand held meter; hand-holding said meter and inserting the probe portion thereof substantially vertically into a body of said fluid in a fluid reservoir to a level such that said heating means and temperature monitoring means sensor in said chamber are substantially immersed in a fluid portion semi-encapsulated in said chamber; and monitoring the output of said temperature monitoring means and recording a maximum temperature reached.

Further preferred features and advantages of the invention will appear from the following detailed description given by way of example of some preferred embodiments illustrated with reference to the accompanying drawings wherein:
Fig. 1 shows a pictorial view of a fluid boiling point meter in accordance with the present invention, and especially intended for sensing the boiling point of automotive brake fluid;
Fig. 2 shows a cross-sectional elevation of the probe portion of the meter of Fig. 1;
Fig. 3 shows the lower part of the probe portion (i.e. below line X-Y) to a larger scale (and greater than actual size);
Fig 4A shows a view in the direction of arrow A in Fig 3, while 4B shows a similar view but for an alternative arrangement of the probe bottom;
Fig 5 shows a view in the direction of arrow A but with the probe bottom wall removed;
Fig 6 shows in basic block form the electronic circuit arrangement of the fluid temperature monitoring means of the meter; and
Fig 7 shows a typical graph of temperature against time for the temperature of heated brake fluid measured by the meter.

Referring to the drawings, a temperature indicating device or meter 1 for measuring the boiling point of automotive brake fluid comprises a main casing 2 housing electronic temperature monitoring equipment and a probe 3 projecting downwardly from the casing 2 for insertion into brake fluid contained in a fluid reservoir as schematically illustrated at 4 and located, for example, in the engine compartment of a motor vehicle. The probe 3 houses a heating element 5 for heating brake fluid while a temperature sensing head 6 of a thermocouple (serving as a mechanical electronic transducer) in the probe feeds heated fluid temperature information to the temperature monitoring equipment in the casing 2.

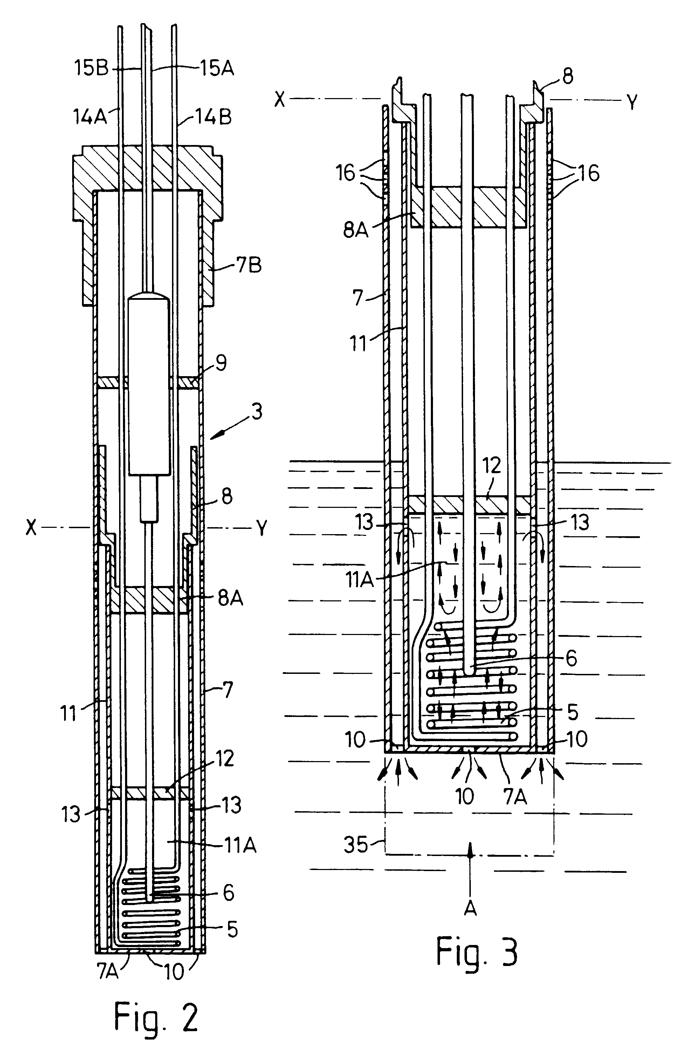

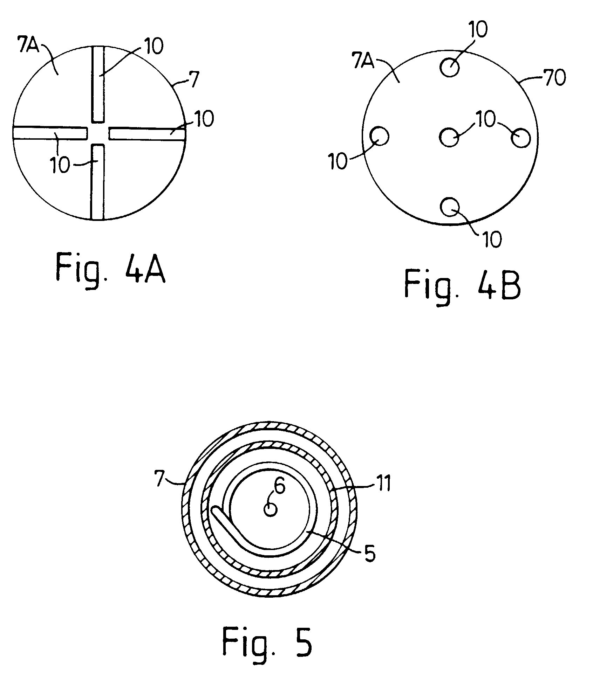

Referring especially to Figs 2 to 5, the probe 3 comprises an outer tubular sheath 7 defining a shroud while the heating element/thermocouple pack is carried by a spaced pair of heat-proof insulator members 8, 9 which in the probe assembled condition bear against the inner wall of the outer sheath 7, and the sheath 7 includes a cap 7B at its upper end which also serves to support the pack. The sheath 7 has a bottom closure wall 7A including a series of fluid apertures 10 (see Figs 4A/4B), and a separate inner tubular casing or sheath 11 is located on a stepped portion 8A of the insulator member 8, the casing 11 having an open bottom which rests on the bottom wall 7A of the sheath 7. The apertures 10 may take the form of slots (Fig 4A) or a series of holes (Fig 4B). An annular fluid space is present between the sheaths 7, 11. The sheaths 7, 11 can be made of suitable material such as stainless steel. A sealing ring 12 (eg of Tufnol (TM)) is housed in the casing 11 and the zone in the casing 11 below this ring 12 defines a housing 11A wherein a portion of fluid to be tested is semi-encapsulated or entrapped, the heating element 5 and the temperature sensing probe 6 being located in this housing 11A. In particular, the heating element 5 is located in the lower half of the housing 11A, and the probe 6 extends into the heart of the element. Initial filling of the housing 11A with fluid is achieved via the apertures 10 and 13. In Fig 4B, the outer holes 10 are preferably aligned with the space between the shrouds 7, 11. Further, the casing 11 includes an annular series of apertures 13 adjacent to the sealing ring 12 whereby fluid can flow between the housing 11A and the annular fluid space between the sheaths 7, 11.

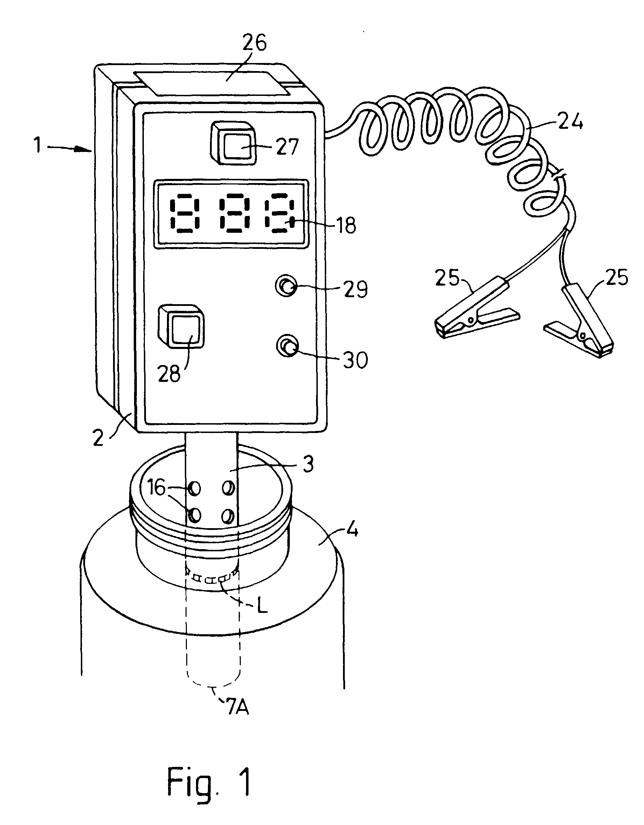

The leads 14A/B and 15A/B for the heating element 5 and thermocouple head 6 respectively extend up through the probe 3. The outer sheath 7 includes a level mark L (Fig 1) to which the probe 3 should be immersed to ensure that the heating element 5 is satisfactorily immersed. Vent holes 16 are provided on the sheath 7.

A suitable circuit for the electronic temperature monitoring equipment is shown in Fig 5. The major item in this circuit is a micro-processor 17 which transmits each monitored temperature value in digital form to a digital display 18 (liquid crystal form). More particularly, a temperature sensing thermocouple 19 of the probe 6 is connected to a closely coupled amplifier and micro-powered thermocouple cold junction compensation chip 20, to obtain most satisfactory results. To convert the output of the matched chips into a form which the micro-processor 17 understands the output from item 20 is fed to an analogue to digital converter 21 (say 10 bit). The processor 17 is driven by an erasable programmable memory (EPROM) 22 and constantly measures and monitors the temperature of the fluid within the housing 11A. The eprom 22 holds the temperature measurement information (program) which is used in sensing the precise boiling point of the fluid and also to generate to BCD (binary coded decimal) code for the display 18. A suitable latch can be present between the EPROM 22 and the processor 17. The processor 17 serves to produce all the information for the various items of the monitoring circuit equipment. Once the equipment senses the fluid boiling point the display 18 will indicate the appropriate temperature value. Additionally, a temperature control switch 5A for the heater 5 receives signals from the micro-processor 17 to halt power to the heater when the measured fluid temperature rises to a predetermined level.

Power for the meter 1 can be taken from the vehicle battery via flexible leads 24 and terminal clips 25 and/or the meter 1 can carry its own battery (eg 12 volts) for example located in housing 26. The casing 2 carries on-off buttons 27, 28 for the power supply to the heating element 5 and to the display equipment respectively, while indicator lamps 29, 30 indicate when the appropriate power via button switches 27, 28 is on, although a single indicator lamp could suffice for both functions.

All the parts of the meter are housed in the casing 2 or in the probe 3 and the meter is a conveniently portable hand-held device.

In operation of the meter 1 to test the quality of brake fluid in a vehicle, the cap of the fluid reservoir housing the brake fluid to be tested is removed and the probe 3 is inserted into the fluid to the level indicated L. Brake fluid flows via the apertures 10, 13 to immerse the heating element 5 and such that a semi-encapsulated body of fluid is located in the housing 11A. Button 27 is now pressed to heat element 5 and consequently cause heating of the encapsulated brake fluid in the housing 11A. With button 28 actuated, the temperature monitoring equipment will monitor the increasing fluid temperature values.

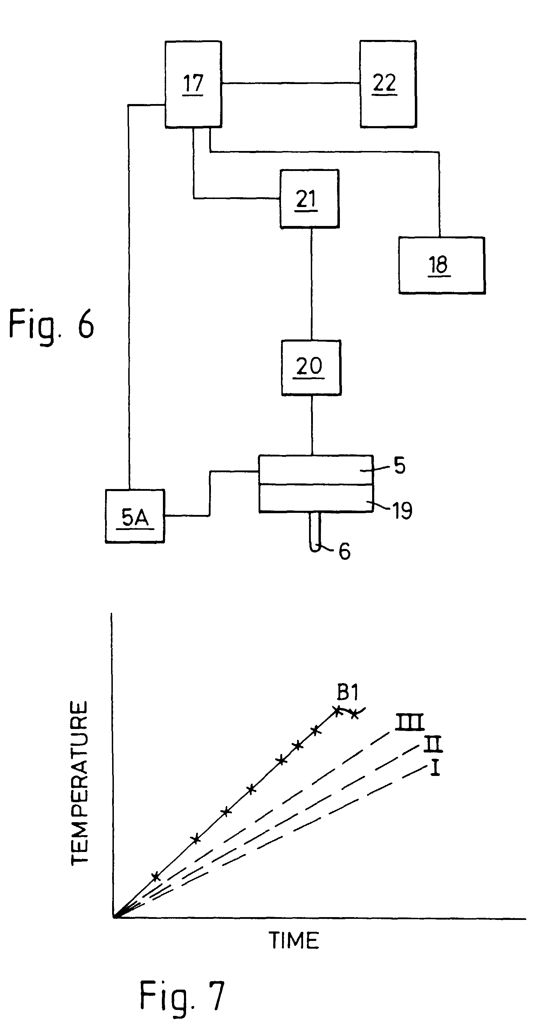

The temperature reading rises to a first maximum or peak (B1 in the graph in Fig 7) then falls slightly before rising again, and subsequently the temperature/time graph will have an oscillating characteristic. The reason for this feature is believed to be due to the fact that, whereas prior to the boiling point the encapsulated fluid in the housing 11A is in a substantially static state, at the on-set of boiling fluid flow paths of a reflux nature are established within the housing 11A, encouraged by the constricted nature of the fluid in the housing and by the location of the heating element 5. During this fluid movement some fluid is discharged via the apertures 10 and 13, although the apertures restrict direct flow of fluid from the housing and the fluid flow paths at boiling will generally be as indicated by the arrows at the bottom end of the probe 3 in Fig 3. The reflex fluid flow results in instantaneous cooling at the temperature sensing zone of the probe 6 causing the temperature reading fall subsequent to the first temperature peak (B1). The highest temperature reached prior to the first temperature drop is taken as the fluid boiling point temperature - in essence this represents the equilibrium reflux boiling point temperature of the fluid. Thus the continuous graph line in Fig 7 is produced from continuous readings from the thermocouple 6 and as will be noted at point B1 there is a temperature drop: point B1 therefore represents the fluid boiling point. The monitoring equipment will have the facility to set the value B1 for reference. Also, it is possible for the monitoring equipment to be arranged so as to equate the measured boiling point as tested to any standard required eg as represented by the dashed lines I, II, etc in Fig 6. Further, ambient temperature can be automatically compensated for and batch tests can be carried out without waiting for cooling. The complete test from start to finish can be carried out very quickly and very conveniently (without the creation of mess or fumes) and, furthermore, other than the brake fluid reservoir cap, nothing has to be removed from the vehicle. The test using the meter 1 should give a tested boiling point reading very close to the correct value ie within 2 or 3 degree centigrade.

At a predetermined elevated temperature the switch 5A is actuated by the micro-processor 17 to halt the supply of electrical power to the heating element 5 thereby avoiding or minimising the risk of formation of noxious brake fluid vapours. The heating element 5 can provide intense local heating in the housing 11A so enabling very rapid testing of the fluid, and this is condusive to the achievement of accurate results. Also, the meter can be used satisfactorily to test liquids where normally there would be the risk of decomposition of the liquid.

Modifications are of course possible. For example a downwardly projecting shroud (indicated by dashed line 35 in Fig 3) could be provided and this would have the advantage of "holding" the external fluid portion adjacent to the slots (apertures) 10 so enabling satisfactory testing even when the probe 3 is moved about in a reservoir of fluid to be tested.

Whereas the meter 1 was described above for testing brake fluid the meter could be used for testing other fluids, especially hydraulic fluids. In particular, transformer fluid could be tested.

## Claims

1. An apparatus for use in indicating the boiling point of fluid especially a hygroscopic fluid such as hydraulic fluid, and comprising a meter (1) of the portable hand held type formed and arranged for insertion of a probe portion (3) thereof substantially vertically into a body of fluid in a fluid reservoir for fluid testing, heating means (5) being provided in the probe portion (3) for heating fluid in which the probe portion (3) is disposed, said meter including temperature monitoring means (6) for monitoring the temperature rise of fluid heated by said heating means for use in indicating the boiling point temperature of the fluid, said probe portion (3) including a casing (11) defining a chamber (11A) located at the lower end of the probe portion (3) so as to be substantially immersed in said body of fluid when said probe portion (3) is inserted thereinto for holding a portion of said body of fluid within the casing (11) said heating means (5) being located in said chamber (11A) and said probe portion (3) having upper and lower fluid port means (10, 13) which exit below a fluid fill level in said chamber (11A) for permitting flow of fluid to and from said chamber (11A) at upper and lower end portions thereof so that a said portion of said body of fluid can be semi-encapsulated in said chamber (11A), and the temperature monitoring means having a sensor (6) disposed in said chamber (11A) so as to be substantially immersed in said semi-encapsulated fluid portion for monitoring the change in temperature thereof when heated by said heating means (5);
characterized in that said probe portion (3) includes a bottom closure wall (7A) defining the lower fluid port means (10) so that the chamber (11A) is substantially enclosed within the probe portion (3), and said upper and lower fluid port means (10, 13) are of a substantially restricted form for substantially preventing flow of fluid to and from said chamber (11A) during heating of said fluid up to its boiling point.

2. An apparatus as claimed in claim 1 wherein said lower aperture means comprises a plurality of spaced apart apertures (10).

3. An apparatus as claimed in claim 2 characterized in that said apertures are in the form of slots or holes (10).

4. An apparatus as claimed in any one of claims 1 to 3, characterized in that an outer shroud (7) surrounds the casing (11) so as to define an outer compartment for holding fluid between the shroud (7) and the casing (11), said outer shroud (7) having fluid aperture means (10) at its lower end.

5. An apparatus as claimed in claim 4, characterized in that the outer shroud (7) has a bottom transverse wall (7A) and the casing (11) has an open lower end resting on said transverse wall, which constitutes said bottom closure wall of the probe portion (3).

6. An apparatus as claimed in any one of claims 1 to 5 characterized in that said second fluid aperture means (13) is disposed above said temperature monitoring means sensor (6).

7. An apparatus as claimed in any one of the preceding claims, characterized in that the monitoring means (6) comprise electronic devices.

8. An apparatus as claimed in claim 7, characterized in that the electronic device includes a micro-processor (17) housed in the meter.

9. An apparatus as claimed in any one of the preceding claims, characterized in that said temperature monitoring means (6) has a thermocouple sensor (19).

10. An apparatus as claimed in claim 9 when dependent on claim 7, characterized in that the thermocouple sensor (19) feeds to a compensation chip and amplifier (20) in turn feeding to an analogue/digital convertor (21) the output from which is supplied to the micro-processor (17).

11. An apparatus as claimed in any one of the preceding claims, characterized in that digital display apparatus (18) is provided to indicate the measured fluid temperature.

12. An apparatus as claimed in claim 8 or claim 10, wherein the micro-processor (17) is linked to a programmable memory (22).

13. An apparatus as claimed in any one of the preceding claims, characterized in that the meter houses an electrical battery for power supply to the heating means (5) and to the temperature monitoring means (6).

14. An apparatus as claimed in any one of the preceding claims, characterized in that electrical cable and connector means (24, 25) are provided to enable the meter to be supplied with electrical power from a vehicle battery.

15. An apparatus as claimed in any one of the preceding claims, characterized in that the probe portion (3) includes at least one ventilation aperture (16).

16. An apparatus as claimed in any one of the preceding claims, characterized in that the probe portion includes a level indicator (1) to facilitate insertion of the probe portion (3) into said body of fluid to be tested to a depth whereat said chamber (11A) is below the surface of said body of fluid.

17. An apparatus as claimed in any one of the preceding claims, characterized in that control means (5A) are provided to halt automatically the power supply to the heating means (5) when the temperature of the fluid portion under test reaches a predetermined level.

18. An apparatus as claimed in any one of the preceding claims, characterized in that heating means (5) comprises a heating element located in a lower zone of the housing (11A) of the probe portion (3), said heating element being of annular form with a central space and in that the temperature monitoring means sensor (6) extends into the central space of the heating element.

19. An apparatus as claimed in any one of the preceding claims, characterized in that the casing (11) comprises a tube, and the upper end of the chamber (11A) is defined by a seal member (12).

20. An apparatus as claimed in any one of the preceding claims, characterized in that insulated spacer means (8, 9) are provided engaging an internal wall of the probe portion (3), said spacer means (8, 9) serving to locate leads of the heating means (5) and of the temperature monitoring means (6).

21. An apparatus as claimed in claim 4 or any one of claims 5 to 20 when dependent on claim 4 characterized in that the shroud (7) extends downwardly below the chamber (11A).

22. An apparatus as claimed in any one of the preceding claims wherein said chamber (11A) is substantially unobstructed so as to permit substantially free circulation of said fluid portion around the heating means (5) and temperature monitoring means (6).

23. A method of determining the boiling point of fluid especially a hygroscopic fluid such as hydraulic fluid, which method comprises the steps of:
providing an apparatus according to claim 1 and comprising a portable hand held meter;
hand-holding said meter (1) and inserting the probe portion (3) thereof substantially vertically into a body of said fluid in a fluid reservoir to a level such that said heating means (5) and temperature monitoring means sensor (6, 19) in said chamber (11A) are substantially immersed in a fluid portion semi-encapsulated in said chamber (11A); and
monitoring the output (18) of said temperature monitoring means (6, 19) and recording a maximum temperature reached.

## Patentansprüche

1. Vorrichtung zur Verwendung des Anzeigens des Siedepunkts von Fluid, insbesondere eines hygroskopischen Fluids, wie beispielsweise Hydraulikfluid, und mit einer tragbaren, mit der Hand gehaltenen Meßeinrichtung (1), die zum im wesentlichen vertikalen Einsetzen eines Fühlerteils (3) hiervon in einen Fluidkörper in einem Fluidreservoir zur Fluidprüfung ausgebildet und angeordnet ist, Heizmitteln (5) im Fühlerteil (3) zur Erwärmung von Fluid, worin der Fühlerteil (3) angeordnet ist, wobei die Meßeinrichtung Temperaturüberwachungsmittel (6) zur Überwachung des Temperaturanstiegs des durch die Heizmittel erwärmten Fluids zur Verwendung bei der Anzeige der Siedepunkttemperatur des Fluids aufweist, wobei der Fühlerteil (3) ein Gehäuse (11) aufweist, wodurch eine Kammer (11A) ausgebildet ist, welche am unteren Ende des Fühlerteils (3) angeordnet ist, um im wesentlichen in den Fluidkörper eingetaucht zu sein, wenn der Fühlerteil (3) darin eingeführt ist, um einen Teil des Fluidkörpers innerhalb des Gehäuses (11) zu halten, wobei die Heizmittel (5) in der Kammer (11A) angeordnet sind und der Fühlerteil (3) obere und untere Fluiddurchlaßmittel (10, 13) aufweist, welche unter einem Fluidfüllstand in der Kammer (11A) austreten, um einen Fluidfluß zu und von der Kammer (11A) am oberen und unteren Endbereich hiervon zu ermöglichen, so daß der Teil des Fluidkörpers in der Kammer (11A) halb eingekapselt sein kann, und wobei die Temperaturüberwachungsmittel einen Sensor (6) aufweisen, welcher in der Kammer (11A) angeordnet ist, um im wesentlichen in dem halb eingekapselten Fluidteil eingetaucht zu sein, um die Änderung der Temperatur hiervon zu überwachen, wenn es durch die Heizmittel (5) erwärmt ist;
dadurch gekennzeichnet, daß der Fühlerteil (3) eine untere Schließwand (7A) aufweist, welche die unteren Fluiddurchlaßmittel (10) ausbildet, so daß die Kammer (11A) innerhalb des Fühlerteils (3) im wesentlichen eingeschlossen ist, und die oberen und unteren Fluiddurchlaßmittel (10, 13) eine im wesentlichen eingeschränkte Form aufweisen, um einen Fluidfluß zu und von der Kammer (11A) während der Erwärmung des Fluids bis zu dessen Siedepunkt im wesentlichen zu verhindern.

2. Vorrichtung gemäß Anspruch 1, bei der das untere Öffnungsmittel eine Mehrzahl beabstandeter Öffnungen (10) aufweist.

3. Vorrichtung gemäß Anspruch 2, dadurch gekennzeichnet, daß die Öffnungen in Form von Schlitzen oder Löchern (10) vorliegen.

4. Vorrichtung gemäß einem der Ansprüche 1 bis 3, dadurch gekennzeichnet, daß eine Außenabdeckung (7) um das Gehäuse (11) herum angeordnet ist, um ein Außenabteil zum Halten von Fluid zwischen der Abdeckung (7) und dem Gehäuse (11) auszubilden, wobei die Außenabdeckung (7) am unteren Ende hiervon Fluidöffnungsmittel (10) aufweist.

5. Vorrichtung gemäß Anspruch 4, dadurch gekennzeichnet, daß die Außenabdeckung (7) eine untere Querwand (7A) aufweist und das Gehäuse (11) ein offenes unteres Ende aufweist, welches sich auf der Querwand befindet, wodurch die untere Schließwand des Fühlerteils (3) ausgebildet ist.

6. Vorrichtung gemäß einem der Ansprüche 1 bis 5, dadurch gekennzeichnet, daß das zweite Fluidöffnungsmittel (13) über dem Temperaturüberwachungsmittelsensor (6) angeordnet ist.

7. Vorrichtung gemäß einem der vorhergehenden Ansprüche, dadurch gekennzeichnet, daß die Überwachungsmittel (6) elekronische Vorrichtungen aufweisen.

8. Vorrichtung gemäß Anspruch 7, dadurch gekennzeichnet, daß die elektronische Vorrichtung einen Mikroprozessor (17) in der Meßeinrichtung aufweist.

9. Vorrichtung gemäß einem der vorhergehenden Ansprüche, dadurch gekennzeichnet, daß das Temperaturüberwachungsmittel (6) einen Thermoelementsensor (19) aufweist.

10. Vorrichtung gemäß Anspruch 9, wenn von Anspruch 7 abhängig, dadurch gekennzeichnet, daß der Thermoelementsensor (19) einen Kompensationschip und einen Verstärker (20) speist, wodurch wiederum ein Analog/Digital-Wandler (21) gespeist ist, dessen Ausgang dem Mikroprozessor (17) zugeführt ist.

11. Vorrichtung gemäß einem der vorhergehenden Ansprüche, dadurch gekennzeichnet, daß eine Digitalanzeigevorrichtung (18) zur Verfügung gestellt ist, um die gemessene Fluidtemperatur anzuzeigen.

12. Vorrichtung gemäß Anspruch 8 oder Anspruch 10, bei der der Mikroprozessor (19) mit einem programmierbaren Speicher (22) verbunden ist.

13. Vorrichtung gemäß einem der vorhergehenden Ansprüche, dadurch gekennzeichnet, daß in der Meßeinrichtung eine elektrische Batterie zur Energiezufuhr zu den Heizmitteln (5) und zu den Temperaturüberwachungemitteln (6) untergebracht ist.

14. Vorrichtung gemäß einem der vorhergehenden Ansprüche; dadurch gekennzeichnet, daß elektrische Kabel- und Verbindungsmittel (24, 25) zur Verfügung gestellt sind, um eine Versorgung der Meßeinrichtung mit elektrischer Energie von einer Fahrzeugbatterie zu ermöglichen.

15. Vorrichtung gemäß einem der vorhergehenden Ansprüche, dadurch gekennzeichnet, daß der Fühlerteil (3) mindestens eine Ventilatoröffnung (16) aufweist.

16. Vorrichtung gemäß einem der vorhergehenden Ansprüche, dadurch gekennzeichnet, daß der Fühlerteil einen Standanzeiger (1) zur Erleichterung des Einführens des Fühlerteils (3) in den Körper des zu testenden Fluids bis zu einer Tiefe, wo die Kammer (11A) unter der Oberfläche des Fluidkörpers ist, aufweist.

17. Vorrichtung gemäß einem der vorhergehenden Ansprüche, dadurch gekennzeichnet, daß Steuermittel (5A) zur Verfügung gestellt sind, um die Energiezufuhr zu den Heizmitteln (5) automatisch anzuhalten, wenn die Temperatur des getesteten Fluidteils einen bestimmten Stand erreicht.

18. vorrichtung gemäß einem der vorhergehenden Ansprüche, dadurch gekennzeichnet, daß das Heizmittel (5) ein Heizelement in einer unteren Zone des Gehäuses (11A) des Fühlerteils (3) aufweist, wobei das Heizelement ringförmig mit einem zentralen Raum ist, und sich der Temperaturüberwachungsmittelsensor (6) in den zentralen Raum des Heizelements erstreckt.

19. Vorrichtung gemäß einem der vorhergehenden Ansprüche, dadurch gekennzeichnet, daß das Gehäuse (11) ein Rohr aufweist und das obere Ende der Kammer (11A) durch ein Dichtelement (12) ausgebildet ist.

20. Vorrichtung gemäß einem der vorhergehenden Ansprüche, dadurch gekennzeichnet, daß isolierte Abstandsmittel (8, 9) in Eingriff mit einer Innenwand des Fühlerteils (3) zur Verfügung gestellt sind, wobei die Abstandsmittel (8, 9) dazu dienen, Leitungen der Heizmittel (5) und der Temperaturüberwachungsmittel (6) aufzunehmen.

21. Vorrichtung gemäß Anspruch 4 oder einem der Ansprüche 5 bis 20, wenn von Anspruch 4 abhängig, dadurch gekennzeichnet, daß sich die Abdeckung (7) nach unten unter der Kammer (11A) erstreckt.

22. Vorrichtung gemäß einem der vorhergehenden Ansprüche, bei der die Kammer (11A) im wesentlichen unblockiert ist, um ein im wesentlichen freies Zirkulieren des Fluidteils um die Heizmittel (5) und die Temperaturüberwachungsmittel (6) zu ermöglichen.

23. Verfahren zur Bestimmung des Siedepunkts von Fluid, insbesondere eines hygroskopischen Fluids, wie beispielsweise Hydraulikfluid, wobei das Verfahren folgende Schritte umfaßt:
Bereitstellung einer Vorrichtung gemäß Anspruch 1 und mit einer tragbaren, mit der Hand gehaltenen Meßeinrichtung;
Halten der Meßeinrichtung (1) mit der Hand und Einführen des Fühlerteils (3) hiervon im wesentlichen vertikal in einen Fluidkörper in einem Fluidreservoir bis zu einem Stand, so daß die Heizmittel (5) und der Temperaturüberwachungsmittelsensor (6, 19) in der Kammer (11A) im wesentlichen in einen Fluidteil eingetaucht sind, welcher in der Kammer (11A) halb eingekapselt ist; und Überwachung des Ausgangs (18) der Temperaturüberwachungsmittel (6, 19) und Aufzeichnung einer erreichten Maximaltemperatur.

## Revendications

1. Appareil destiné à indiquer le point d'ébullition d'un fluide, de manière spécifique d'un fluide hygroscopique tel qu'un fluide hydraulique, et comprenant un dispositif de mesure (1) du type portatif formé et agencé pour l'insertion de sa partie formant sonde (3) sensiblement à la verticale dans le corps d'un fluide dans un réservoir de fluide pour le contrôle du fluide, des moyens de chauffage (5) étant prévus dans la partie formant sonde (3) pour chauffer le fluide dans lequel la partie formant sonde (3) est disposée, ledit dispositif de mesure comprenant des moyens de contrôle de température (6) pour contrôler la montée en température du fluide chauffé par lesdits moyens de chauffage à utiliser pour indiquer la température du point d'ébullition du fluide, ladite partie formant sonde (3) comprenant un boîtier (11) définissant une chambre (11A) située au niveau de l'extrémité inférieure de la partie formant sonde (3) de façon à être sensiblement immergée dans ledit corps de fluide lorsque ladite partie formant sonde (3) est insérée dans ce dernier pour maintenir une partie dudit corps de fluide à l'intérieur du boîtier (11), lesdits moyens de chauffage (5) étant situés dans ladite chambre (11A) et ladite partie formant sonde (3) ayant des moyens formant orifices supérieurs et inférieurs de fluide (10, 13) qui sortent au-dessous d'un niveau de remplissage de fluide dans ladite chambre (11A) pour permettre l'écoulement du fluide vers et en provenance de ladite chambre (11A) au niveau des parties d'extrémités supérieure et inférieure de cette dernière de sorte qu'une dite partie dudit corps de fluide peut être semi-encapsulée dans ladite chambre (11A), et les moyens de contrôle de température ayant un capteur (6) disposé dans ladite chambre (11A) de façon à être sensiblement immergé dans ladite partie de fluide semi-encapsulée pour contrôler le changement de température de ce dernier quand il est chauffé par lesdits moyens de chauffage (5) ;
caractérisé en ce que ladite partie formant sonde (3) comprend une paroi de fermeture de fond (7A) définissant les moyens formant orifices inférieurs de fluide (10) de sorte que la chambre (11A) est sensiblement enfermée à l'intérieur de la partie formant sonde (3), et lesdits moyens formant orifices supérieurs et inférieurs de fluide (10, 13) ayant une forme sensiblement limitée pour empêcher sensiblement un écoulement de fluide vers et en provenance de ladite chambre (11A) pendant le chauffage dudit fluide jusqu'à son point d'ébullition.

2. Appareil selon la revendication 1, dans lequel lesdits moyens formant ouvertures inférieures comprennent une pluralité d'ouvertures espacées (10).

3. Appareil selon la revendication 2, caractérisé en ce que lesdites ouvertures ont la forme de fentes ou trous (10).

4. Appareil selon l'une quelconque des revendications 1 à 3, caractérisé en ce qu'une enveloppe extérieure (7) entoure le boîtier (11) de façon à définir un compartiment extérieur pour maintenir le fluide entre l'enveloppe (7) et le boîtier (11), ladite enveloppe extérieure (7) ayant des moyens d'ouvertures de fluide (10) au niveau de son extrémité inférieure.

5. Appareil selon la revendication 4, caractérisé en ce que l'enveloppe extérieure (7) possède une paroi transversale de fond (7A) et en ce que le boîtier (11) possède une extrémité inférieure ouverte reposant sur ladite paroi transversale, qui constitue ladite paroi de fermeture de fond de la partie formant sonde (3).

6. Appareil selon l'une quelconque des revendications 1 à 5, caractérisé en ce que lesdits seconds moyens formant ouvertures de fluide (13) sont disposés au-dessus dudit capteur de moyens de contrôle de température (6).

7. Appareil selon l'une quelconque des revendications précédentes, caractérisé en ce que les moyens de contrôle (6) comprennent des dispositifs électroniques.

8. Appareil selon la revendication 7, caractérisé en ce que le dispositif électronique comprend un microprocesseur (17) logé dans le dispositif de mesure.

9. Appareil selon l'une quelconque des revendications précédentes, caractérisé en ce que lesdits moyens de contrôle de température (6) possèdent un capteur à thermocouple (19).

10. Appareil selon la revendication 9 lorsqu'elle dépend de la revendication 7, caractérisé en ce que le capteur à thermocouple (19) alimente un amplificateur et puce de compensation (20), alimentant à son tour un convertisseur analogique-numérique (21) dont la sortie est délivrée au microprocesseur (17).

11. Appareil selon l'une quelconque des revendications précédentes, caractérisé en ce qu'un appareil d'affichage numérique (18) est prévu pour indiquer la température mesurée du fluide.

12. Appareil selon la revendication 8 ou la revendication 10, dans lequel le microprocesseur (17) est relié à une mémoire programmable (22).

13. Appareil selon l'une quelconque des revendications précédentes, caractérisé en ce que le dispositif de mesure loge une batterie électrique pour l'alimentation en courant des moyens de chauffage (5) et des moyens de contrôle de température (6).

14. Appareil selon l'une quelconque des revendications précédentes, caractérisé en ce que des moyens formant câble électrique et connecteur (24, 25) sont prévus pour permettre au dispositif de mesure d'être alimenté en courant électrique à partir d'une batterie de véhicule.

15. Appareil selon l'une quelconque des revendications précédentes, caractérisé en ce que la partie formant sonde (3) comprend au moins une ouverture de ventilation (16).

16. Appareil selon l'une quelconque des revendications précédentes, caractérisé en ce que la partie formant sonde comprend un indicateur de niveau (1) pour faciliter l'insertion de la partie formant sonde (3) dans ledit corps de fluide à contrôler jusqu'à une profondeur au niveau de laquelle ladite chambre (11A) est au-dessous de la surface dudit corps de fluide.

17. Appareil selon l'une quelconque des revendications précédentes, caractérisé en ce que des moyens de commande (5A) sont prévus pour suspendre de façon automatique l'alimentation en courant vers les moyens de chauffage (5) lorsque la température de la partie de fluide en train d'être contrôlée atteint un niveau prédéterminé.

18. Appareil selon l'une quelconque des revendications précédentes, caractérisé en ce que les moyens de chauffage (5) comprennent un élément de chauffage situé dans une zone inférieure du logement (11A) de la partie formant sonde (3), ledit élément de chauffage étant de forme annulaire avec un espace central et en ce que le capteur des moyens de contrôle de température (6) s'étend dans l'espace central de l'élément de chauffage.

19. Appareil selon l'une quelconque des revendications précédentes, caractérisé en ce que le boîtier (11) comprend un tube, et l'extrémité supérieure de la chambre (11A) est définie par un élément formant joint (12).

20. Appareil selon l'une quelconque des revendications précédentes, caractérisé en ce que des moyens formant dispositifs d'espacement isolés (8, 9) sont prévus pour mettre en prise une paroi interne de la partie formant sonde (3), lesdits moyens formant dispositifs d'espacement (8, 9) servant à installer des conducteurs des moyens de chauffage (5) et des moyens de contrôle de température (6).

21. Appareil selon la revendication 4 ou selon l'une quelconque des revendications 5 à 20 lorsqu'elle dépend de la revendication 4, caractérisé en ce que l'enveloppe (7) s'étend vers le bas au-dessous de la chambre (11A).

22. Appareil selon l'une quelconque des revendications précédentes, dans lequel ladite chambre (11A) est sensiblement non encombrée de façon à permettre une circulation sensiblement libre de ladite partie de fluide autour des moyens de chauffage (5) et des moyens de contrôle de température (6).

23. Procédé de détermination du point d'ébullition d'un fluide, de manière spécifique un fluide hygroscopique tel qu'un fluide hydraulique, lequel procédé comprend les étapes suivantes :
la présence d'un appareil selon la revendication 1 et comprenant un dispositif de mesure portatif ;
la prise en main dudit dispositif de mesure (1) et l'insertion de sa partie formant sonde (3) sensiblement à la verticale dans le corps dudit fluide dans un réservoir de fluide jusqu'à un niveau tel que lesdits moyens de chauffage (5) et ledit capteur des moyens de contrôle de température (6, 19) dans ladite chambre (11A) soient sensiblement immergés dans une partie de fluide semi-encapsulée dans ladite chambre (11A) ; et
le contrôle de la sortie (18) desdits moyens de contrôle de température (6, 19) et l'enregistrement de la température maximale atteinte.
